# EUROPEAN PATENT APPLICATION

(11) **EP 3 391 808 A1**
(43) Date of publication of application: **24.10.2018**
(21) Application number: 17166755.3
(22) Date of filing: 18.04.2017
(51) Int. Cl.: A61B 5/00, A61B 5/06

(54) **METHOD AND SYSTEM FOR DETECTING MIGRATION OF MEDICAL INSERTS OR IMPLANTS**

(71) Applicant: Koninklijke Philips N.V., 5656 AE Eindhoven (NL)
(72) Inventor: VISWESWARA, Ashoka Sathanur, 5656 AE Eindhoven (NL); WORTELBOER, Pippinus Maarten Robertus, 5656 AE Eindhoven (NL); MEFTAH, Mohammed, 5656 AE Eindhoven (NL); JOHNSON, Mark Thomas, 5656 AE Eindhoven (NL)
(74) Representative: de Haan, Poul Erik

(57) **Abstract**

A system is provided for determining the position of an implanted device (10) in a human or animal body relative to a probe (14). In one device, a reference signal is generated and it is received in the other device. A phase angle or an amplitude attenuation or difference between the reference signal and the received signal is used to determine the proximity of the probe to the implanted device. The approach makes use of body channel sensing by which the frequencies at which the body behaves as a waveguide are employed.

## Description

### FIELD OF THE INVENTION

This invention relates to a method and system for detecting migration of medical inserts or implants.

### BACKGROUND OF THE INVENTION

Medical inserts or implants are known to be susceptible for migration and may move around a patient's body, causing serious, sometimes life threatening, injuries and significant pain.

There have been several academic studies and medical case reports of an implanted or inserted medical device migrating inside the human body after its insertion. Examples where a migration issue has been identified include an insertable cardiac monitor, dental implants, transvaginal mesh devices, breast implants, and a contraceptive subcutaneous device in the pulmonary artery of the patient.

Typically, the detection of device migration takes place only a period of time after the original operation (when the device is placed inside the body) when the patient complains of pain or other abnormal side effects. The method for detection usually involves performing expensive body scans using complex imaging machines in a hospital. If the patient can gain knowledge of the device migration much earlier than by sensing the reaction of the body to the migration, the patient can take preventative steps before the situation worsens.

This requires that a patient carries a device which he/she uses to periodically check whether or not the implanted device has migrated from its original position. At present, there is no such device available.

There is therefore a need for a system which can monitor the position of an implanted or inserted device which is accurate and convenient for the user.

### SUMMARY OF THE INVENTION

The invention is defined by the claims.

Examples in accordance with a first aspect of the invention provide a system for determining the position of an implanted device in a human or animal body relative to a probe, wherein the implanted device comprises an implanted device 10 MHz to 200 MHz RF transmitter or receiver, and the probe comprises a probe 10 MHz to 200 MHz RF receiver or transmitter, wherein the system comprises:
a controller which is adapted to determine a phase angle and/or an amplitude attenuation or difference between a reference signal and a received signal which has passed through the body and thereby determine the proximity of the probe to the implanted device.

In this system, a proximity i.e. distance measurement is made between a probe and an implanted device, based on signal attenuation and/or phase changes resulting from signal propagation. In either case, a low cost unobtrusive way is enabled to detect implant (or other medical insert) device migration. The approach makes use of body channel sensing by which the frequencies at which the body behaves as a waveguide are employed. By measuring a phase angle change and/or attenuation level of a transmitted body channel RF signal to a remote detection device, the distance between the devices can be determined.

Note that the term "10 MHz to 200 MHz RF receiver" or "10 MHz to 200 MHz transmitter" means that the frequency used is somewhere within that range.

The determining system compares a reference signal with a related received signal from the other device. The determining system may be the probe device or the implanted device, and the received signal comes from the respective other device.

The controller may be adapted to determine a phase angle, in which case the implanted device comprises an implanted device clock and the probe comprises a probe clock. The system then comprises a clock synchronization system for synchronizing the probe clock with the implanted device clock. This clock synchronization ensures that a phase difference can be translated to a propagation distance. The two devices have bidirectional communication between them to enable the clock synchronization to be performed. This communication is for example implemented in addition to the RF system for the body channel sensing signal. The RF system for proximity measurement is selected to optimize performance based on the signal transmission through the body (at 10 MHz to 200 MHz for example 10 MHz to 150MHz), whereas the communication system may use any standard wireless communications protocol, such as Bluetooth (at around 2.4 GHz). The reference signal is then a signal which has not passed through the body, for example generated at the probe, and it is then compared in phase with a signal which has been transmitted through the body.

The system may comprise a database which stores mappings between phase angles and/or amplitude attenuations and proximity distances. This database may be established by a calibration routine, and it enables distance measurements to be obtained with minimal calculation.

An alarm system may be provided for generating an alarm if a detected distance exceeds a threshold value which depends on a proximity value corresponding to an initial placement of the implanted device. Thus, if the implanted device has moved from its initial position by more than a threshold amount, an alarm may be created. This may provide an alert before the subject is aware of a potential issue.

The system may further comprise a position determining system for determining an implanted device position based on a set of at least three proximity determinations each associated with a pre-determined probe position. The at least three proximity determinations may be adapted to be carried out simultaneously from a probe having a set of probe RF transceivers, or they may be carried out in sequence. By using at least three known probe locations (on or in the body), migration detection may be based on a triangulation method, which enables not only a proximity distance to be determined but also an actual position.

In one set of examples, the position determination system is part of the probe. Thus, the proximity calculations are carried out at the probe, so the implanted device may be as simple as possible.

In another set of examples, the system is part of the implanted device, and the system further comprises a wireless communication system for communicating the determined proximity so that it can be picked up externally to the body. This communication system is for example the same one used for communication between the devices, such as for establishing clock synchronization.

In one set of examples, the probe is an external probe. Thus, there is an external probe to be applied to the skin and an internal implanted device. The system may then further comprise a marker for application to the skin to identify an initial position closest to the implanted device. This provides a fixed reference point from which proximity measurements are made.

In another set of examples, the probe is also an implanted device. It may for example be fixed to a location where it cannot migrate, for example attached to bone. This avoids the need for the subject to place the probe accurately. Instead, an external communication device may be used to instigate communication between the probe and the implanted device being monitored, and the results are then reported to the external communication device. The implanted probe may have multiple transceivers at multiple fixed positions for the triangulation approach mentioned above.

The invention also provides an implant system, comprising:
an implantable device which comprises an implanted device 10 MHz to 200 MHz RF transmitter or receiver;
a probe, which comprises a probe 10 MHz to 200 MHz RF receiver or transmitter; and
a system as defined above provided at the implantable device or at the probe.

Examples in accordance with another aspect of the invention provide a method for determining the position of an implanted device in a human or animal body relative to a probe, wherein the implanted device comprises an implanted device 10 MHz to 200 MHz RF transmitter or receiver, and the probe comprises a 10 MHz to 200 MHz RF receiver or transmitter, wherein the method comprises:
generating a reference signal at one of the implanted device and the probe; and
at the other of the implanted device and the probe determining a phase angle and/or an amplitude attenuation or difference between the reference signal and a received signal; and
thereby determining the proximity of the probe to the implanted device.

In one approach, the implanted device comprises an implanted device clock and the probe comprises a probe clock. The probe clock is synchronized with the implanted device clock, and a phase angle is used to determine the proximity. Stored mappings may be used to translate between phase angles and/or amplitude attenuations and proximity distances.

The computations may be implemented in software.

### BRIEF DESCRIPTION OF THE DRAWINGS

Examples of the invention will now be described in detail with reference to the accompanying drawings, in which:
Figure 1 shows an example of a system in accordance with an example;
Figure 2 shows a first human body model for signal transmission inside the body;
Figure 3 shows a second human body model for signal transmission inside the body;
Figure 4 shows plots of a signal generated by a transmitter and two received signals at different distances;
Figure 5 shows an example of the transmitter architecture in more detail;
Figure 6 shows an example of the receiver architecture in more detail;
Figure 7 shows a distance determination method;
Figure 8 shows how multiple detection locations may be used to triangulate a position of the implanted device; and
Figure 9 shows how the multiple detection locations of Figure 8 may be formed as a wearable sensor module.

### DETAILED DESCRIPTION OF THE EMBODIMENTS

The invention provides a system for determining the position of an implanted device in a human or animal body relative to a probe. In one device, a reference signal is generated and it is received in the other device. A phase angle and/or an amplitude attenuation or difference between the reference signal and the received signal is used to determine the proximity of the probe to the implanted device. The approach makes use of body channel sensing by which the frequencies at which the body behaves as a waveguide are employed. Note that the term "implanted device" is used to indicate any electronic device for insertion into the body.

There have been several academic works which describe an electrical field model of the human body. The model shows the frequency response of the human body as a signal transmission medium. It is measured by capacitive coupling of an electrical signal of certain frequency and amplitude at one point on the human body. The received signal measured at a different point on the body by a sensor is analyzed for various transmitter frequencies and various distances (and body locations) between the transmitter and the sensor.

For example, Namjum Cho, e. (2007). The Human Body Characteristics as a Signal Transmission Medium for Intrabody Communication; IEEE Transactions on Microwave Theory and Techniques, suggests a near-field coupling model of the human body by modelling the human body into three cylinders, two for the arms and one for the human torso. The arms and the torso are then modelled as distributed RC network.

From measurements taken, a linear relationship in the attenuation of the signal from a transmitter towards a receiver is observed as the receiver is placed at varying distances from the transmitter, in particular when the frequency used is in a preferred range of 10 MHz to 150MHz. At a frequency lower than 10 MHz, the signal wavelength is much larger than the size of the human body and the electric field around the human body is almost uniform (though varying in time), which means its phase has almost the same value everywhere on the body at some point in time. In this condition, the time-varying electric field around the human body can be regarded as a quasi-static field. As a consequence, these frequencies cannot be used for on-body localization.

On the other hand, at frequencies greater than 10 MHz up to 200 MHz, the body behaves like a waveguide for the RF signal at such frequencies, since the signal wavelength is comparable to the human body dimensions. As a consequence, the electrical signal attenuates as the signal propagates through the human body. This phenomenon helps in calculating the distance of a sensor from a transmitter based on signal attenuation and phase change measurements.

This body channel sensing is for example employed in Zhang, Y. e. (2016). SkinTrack: Using the Body as an Electrical Waveguide for Continuous Finger Tracking on the Skin. Proceedings of the 2016 CHI Conference on Human Factors in Computing Systems*.* In this system, a continuous finger tracking technology is proposed in the form of a wearable system that enables continuous touch tracking on the skin, in particular so that a touch input area becomes larger than the area of a screen of a wrist mounted device.

The system consists of a ring, which emits a continuous high-frequency AC signal, and a sensing wristband with multiple sensor electrodes. Due to the phase delay inherent in the high frequency AC signal propagating through the body, a phase difference can be observed between pairs of electrodes. The system measures these phase differences to compute a 2D finger touch coordinate/location. The resolution is about 7mm.

When an 80MHz RF signal is used, the wavelength of the electromagnetic wave generated is around 91cm. This results in phase angle difference of 4°/cm for one single cycle of the wave. If the localization is performed within one wavelength of the RF signal which is 91 cm, then one can uniquely locate the position of the transmitter with respect to two sensors by measuring the phase angle difference between two received signals.

This approach needs multiple electrodes which need to be placed quite apart for the localization principle to work. This puts restrictions on the size of the detection device which (for an external device) would ideally be only a small patch on the skin surface to continuously detect device migration.

The system of the invention enables only a single electrode pair to be used in the detection (probe) device so that the dimensions of the detection device can be kept small.

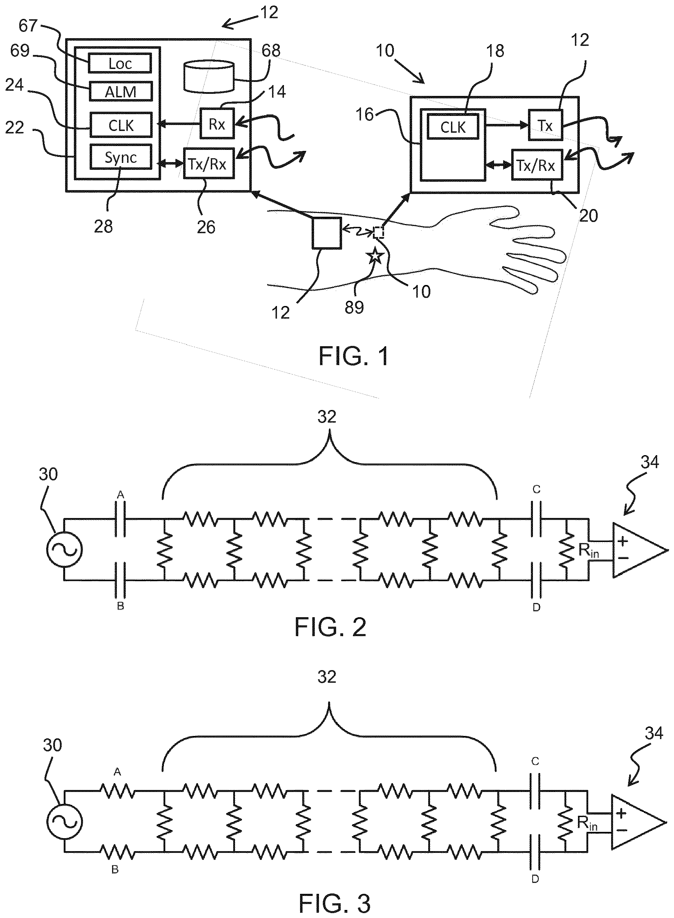

Figure 1 shows an example of a system in accordance with an example.

The overall system comprises an implanted device 10 in a human (or animal) body and a probe 12.

The system relies on the transmission of a signal from one device to the other through the body tissue, and an analysis of the effect of the body tissue transmission. One device functions as a transmitter of the signal and the other device functions as the receiver. This signal is at an RF frequency for body channel sensing as explained above.

In the example shown in Figure 1, the implanted device 10 is the transmitter and it has an RF transmitter 12. The probe is the receiver and it has an RF receiver 14. These may both be implemented as transceivers. In the configuration of Figure 1, the probe implements the position determining system. The system configuration with the probe as the receiver and the implanted device as the transmitter will be described in more detail below, but the opposite implementation is possible.

The implanted device in this example comprises a controller 16 with implanted device clock 18. There is also a communications transceiver 20 to enable communication between the probe and the implanted device - independent of the body channel sensing signal.

The probe in this example comprises a controller 22 with probe clock 24. There is also a communications transceiver 26 to enable communication between the probe and the implanted device.

In the example of Figure 1, the position determining system is implemented in the probe. The clock 24 is used by the controller to implement a reference signal generator which is used for the position determination.

The controller 22 is adapted to determine a phase angle and/or an amplitude attenuation between the reference signal and a received signal which has passed through the tissue, and thereby determine the proximity of the probe to the implanted device. As explained below, one example of how to implement phase angle determination is for the controller 22 to implement a synchronization function, show as sync circuit 28. Alternatively, amplitude measuring circuitry may be used to measure an amplitude attenuation, without needing clock synchronization.

The RF transmitter and receiver operate in the frequency range suitable for body channel sensing. The frequency range is between 10 MHz and 400 MHz, more preferably 10 MHz to 150 MHz. The implanted device has two electrodes which capacitively couple the signal to the human body.

The communications transceivers 20, 26 for example operate using Bluetooth, ultra wideband (UWB) communications, optical communications or any other suitable communications protocols.

In all examples, the invention makes use of signal transmission through the body and comparison with a signal which has not been transmitted through the body.

Figure 1 shows some optional addition features of the position determining system which are discussed further below. These include a location system 67 for detecting location by triangulation as well simply distance measurement, and an alarm system 69 for providing an alarm in the event to insert migration. They are shown as part of the controller 22. Figure 1 also shows a database 68 used for interpreting the measured phase and/or amplitude information. Finally, a marker 89 is also discussed below.

Figure 2 shows a human body model for signal transmission inside the body for such body channel sensing signal frequencies. A signal source is shown as 30 with two electrodes, which couple through capacitances to the conductive body tissue 32, which is represented by a ladder network of resistances. There is capacitive coupling to the receiver amplifier 34. Figure 2 is a model where the transmitter and the receiver are located on the surface of the human body and Figure 3 is a model where the transmitter is located inside the human body and the receiver is located on the surface. In Figure 3, the signal source 30 couples to the conductive body tissue through resistances. Depending on the location of the implant within the body, one of the two models can be used to model the transmission behavior of the RF signal.

A first approach based on measuring a phase delay will first be described. The approach requires only a single pair of electrodes in both the implanted device as well as in the probe.

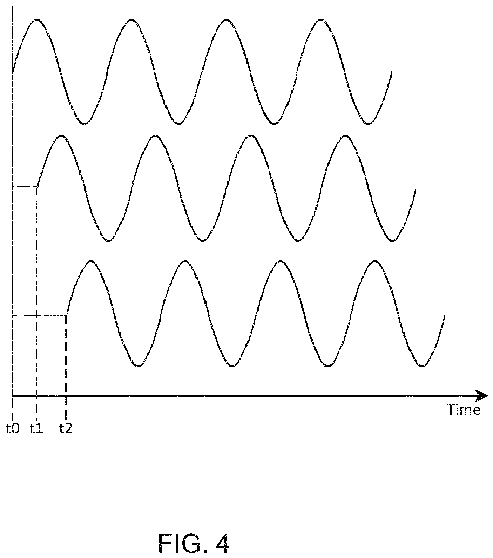

Figure 4 shows as the top plot the signal generated by the transmitter, which is in the implanted device in the example above. The signal is coupled to the body at time t0. After a certain time t1, the waveform in the middle plot reaches the receiver. By determining the phase angle difference between the original signal and the received signal information on the time difference t1-t0 is obtained which corresponds to time it takes for the transmitted wave to reach the receiver. This directly relates to the distance between the transmitter (in this example the implanted device) and the receiver (in this example the external probe).

Similarly if the receiver is placed further apart from the transmitter, the receiver would receive the waveform shown in the bottom plot and the time difference t2-t0 provides the information on how far the receiver is located with respect to the transmitter.

This technique requires the internal clocks 18, 24 of the transmitter (implanted device) and the receiver (probe) to be fully synchronized.

To achieve this synchronization, the separate RF communications system 20, 26 is used. The communications system is first used to synchronize the internal clocks of both the transmitter and the receiver, and this implemented by the sync unit 28. If the internal clocks are not synchronized, then an additional unknown phase angle difference will exist between the two clocks making the distance measurement inaccurate.

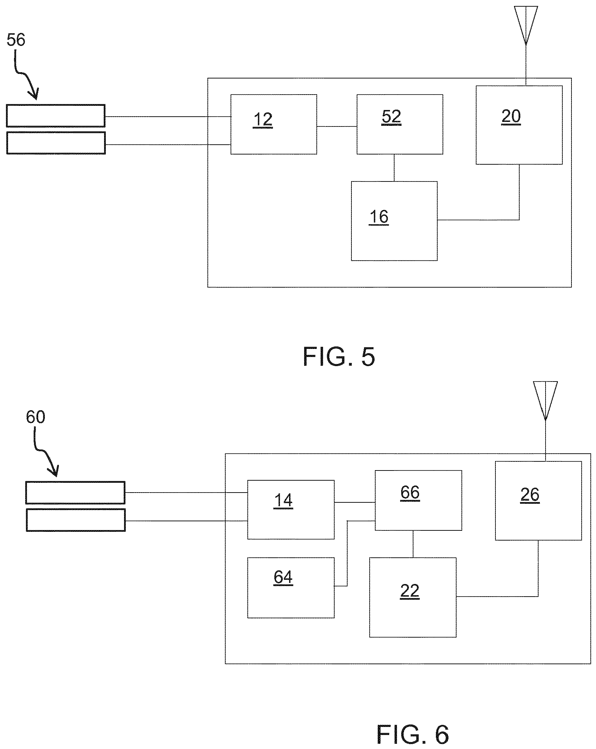

Figures 5 and 6 show an example of the transmitter and receiver architecture respectively in more detail for the phase angle measurement approach. The same reference numbers are used as in Figure 1 for the same components.

Figure 5 shows an example of the transmitter architecture. The transmitter 12 of Figure 1 is driven by a signal generator 52 which is controlled by the controller 16, and it comprises a voltage boosting driver circuit which delivers the RF body channel sensing signal to a pair of transmit electrodes 56.

Figure 6 shows an example of the receiver architecture. There is a pair of receive electrodes 60 which connect to the receiver 14 which is in the form of an analog front end. A signal generator 64 provides a reference signal, and the received signal and reference signal are provided to a unit 66 for phase angle determination. The synchronization function is implemented in the two controllers 16, 22 using communication via the transceivers 20, 26.

The position determination may be used to detect if the implanted device has moved away from the external detection device by more than a threshold. Even though the phase angle difference gives an absolute measure of the distance between the implanted device and the probe, there could be other signal delays inside the electronic circuitry which need to be compensated and this can be achieved by a calibration approach.

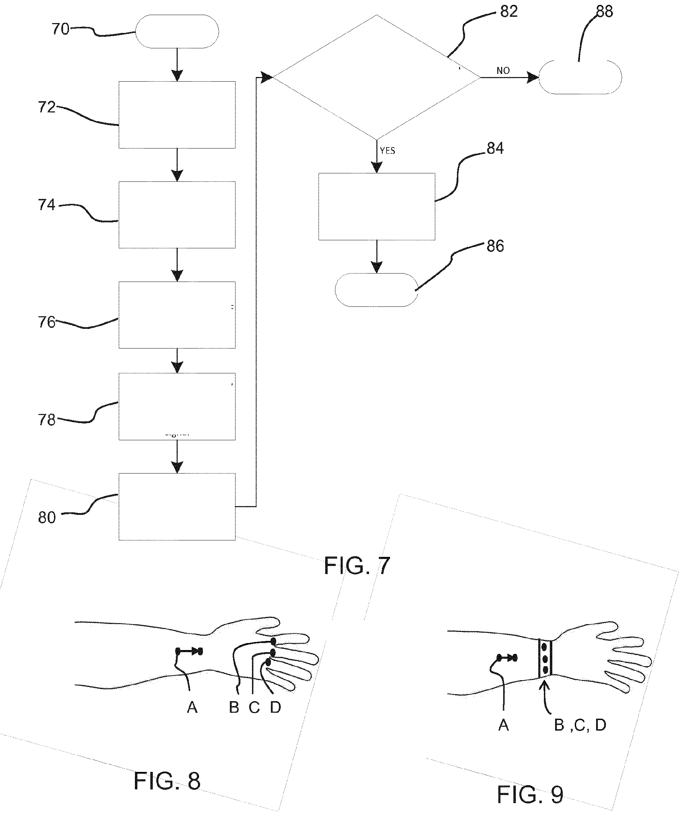

Figure 7 shows a position determination method based on phase measurement.

In general, in a first phase, an external detection device establishes communication with the implanted device using traditional wireless communication such as Bluetooth, UWB, optical communication or other methods and the internal clock of both the devices are synchronized. In the next phase, the implanted device sends a burst of RF signal in a preferred frequency range of 10 MHz- 150 MHz through the human body while the external detection device is held close to the human body (touching the patient body) at a pre-determined location. The phase angle difference between the received signal from the implanted device and an internally generated reference signal at the external detection device is measured.

During the insertion of the implanted device, a calibration step is also performed (not shown) where the phase angle difference between the transmitted signal and the sensed signal is mapped to a known distance between the transmitter and the receiver and this information is stored in the controller of the position determination system in the form of a look-up table or other database. Such a database is shown as 68 in Figure 1.

In Figure 7, the method starts in step 70. In step 72, the transmitter and receiver establish communication. In step 74, the transmitter and receiver synchronize their internal clocks.

In step 76, the transmitter then sends out the body channel sensing signal, typically in the RF range 10 MHz to 150MHz.

In step 78, the receiver receives the signal, and performs a phase comparison with the synchronized internal signal.

In step 80, the controller of the position determining device converts the phase angle difference to a distance measure using a look-up table.

In step 82, an alarm system is implemented for generating an alarm if a detected proximity exceeds a threshold value. This alarm system is shown in Figure 1 as unit 69. If the distance exceeds a threshold, an alarm is generated in step 84 and the process ends in step 86. If the distance does not exceed a threshold, the process ends in step 88. The threshold distance may for example depend on a proximity value corresponding to an initial placement of the implanted device.

To perform accurate localization, various methods are possible.

In one example, after the insertion of implanted device, a device scan is performed where the external probe is used to locate the original placement of the implanted device. This may be achieved by moving the probe on the body near the insertion of the implanted device until the phase angle measured is minimum. This minimum phase angle difference is translated into a distance measure using a lookup table. After locating the minimum phase angle point, a marker is attached to the location, for example as shown in Figure 1 as marker 89. The marker chosen is such that it can be identified easily after weeks or months from the implant insertion date.

In many cases, the position will be associated with scar tissue created during the insertion process. One method for this is to use a patch permanently on the body to identify the location where the implanted device was inserted. Another method is to have a marker on the body surface to identify the original implant position. Any method may be used in which the original position of the implanted device is identified.

This initial step functions as a calibration phase.

During routine periodic check-ups to be conducted by the patient who has the implanted device, the patient first locates the minimum phase angle marker on his/her body and places the probe on the marker.

The phase angle difference between the implanted device and the probe is then computed using the method of Figure 7. During each routine periodic check-up, the marker on the body is refreshed if required so that the marker can be easily detected during the next periodic check.

This method requires a known probe position to be employed. It is also possible to provide migration detection without knowing the initial implant location. With reference to Figure 8, after inserting the implant in a medically preferred initial body location (A), the probe may be placed sequentially on 3 or more preferred body locations (B, C and D) which can be easily found in the future. These for example comprise the knuckles for an implant in the wrist, as shown in Figure 8. In particular, in order to have accurate measurements, these locations should not be far away from the initial implant location.

Reference measurements are then performed. These measurements can then be saved in the probe and used as 'zero migration' reference measurements.

When future measurements are taken, possible migration of the implant (as shown in Figure 8) can be identified easily by repeating the measurements and comparing them against the 'zero migration' reference measurements. Triangulation methods can be used to combine three or more measurements performed at various locations on the body to accurately find the location of the insert before and after migration. Thus, the system may include a location determining system (shown as unit 67 in Figure 1) for this location function based on triangulation.

To prevent false measurements (a false positive measurement will report that there was an alert when the implant didn't in fact migrate, and a false negative will not report that an implant has migrated when in fact it has) due to skin stretching, limb bending, etc., it is important that the body, at the location of the implant and the probe, is kept stable in a standardized location and posture. For example, the user may be required to hold his/her arm on a flat horizontal surface during the measurements.

The multiple external measurements (B, C and D) may be integrated into a single external device such as a watch-like solution as shown in Figure 9.

In the examples above, the probe is an external device. However, it may also be part of an implanted device. Thus, both the transmitter and receiver of the body channel sensing signal are attached to devices that are implanted at different locations. The distance between the transmitter and receiver is measured in a similar way to that explained above.

The trigger to perform a measurement can be generated either by the user by sending a signal to one of the devices with a special purpose (low-cost) external device, or by the implanted device itself using a clock or even an internally measured body signal. As the relative distance between the two implanted devices changes, the last measurement will differ from the previous one(s) and depending on the amount of change a signal is issued to the user. This signal can be directly noticeable (such as audible, tangible) or otherwise sent to an external device that transforms it into such a signal.

If an external device is used that combines a position determining system as well as a communications system, it can communicate with both implanted devices independently (and thus have migration information reported to it) but also individual migration can be detected by the external device itself using the approach described above.

When one of the internal devices is attached to a stable and well-defined body structure like a bone, a built-in reference position is obtained. Any change in distance between the migration-sensitive and the fixed reference device is detected (although migration that consists of moving along a circle around the reference device will not be detected).

With even more internal devices, a network of transmitting and receiving stations can be set up and distance changes between any transmitting and receiving devices can be detected straightforwardly.

The examples above are based on phase comparisons. This approach works only if the internal clocks of the transmitter (e.g. implanted device) and the receiver (external detection probe) are fully synchronized.

To avoid the need for this synchronization, another technique may be used which again requires only a single pair of electrodes in both the implanted device as well as in the external detection device. The technique for detecting the distance between the implanted device and the external detection device uses signal amplitude measurements.

The transmitter generates a signal which is coupled to the body as explained above. After a certain time, the transmitted wave reaches the receiver. The amplitude difference between the transmitted wave and the received wave may be measured to provide the information on distance between the transmitter (e.g., the implanted device) and the receiver (e.g. the external probe).

Even though the amplitude difference gives an absolute measure of the distance between the transmitter and the receiver, there could be other factors which need to be compensated.

To address this issue, during the insertion of the implanted device, a similar calibration step to that described above may be performed where the amplitude difference between the transmitted signal and the sensed signal is mapped to a known distance between the transmitter and the receiver, and this information is stored in the controller of the position determination system in the form of a look-up table.

The localization techniques explained above may be used to accurately map the implanted device both during insertion (for the calibration phase) and after migration during the check-up phase.

The amplitude measurement approach does not need clock synchronization. Thus, it also does not need communication between the probe and the insert as part of the signal measurement. A communication system may however be provided for the probe to communicate with the insert. For example, the probe may be used to instruct the insert to issue a body channel sensing signal when position detection is desired. The amplitude of the received signal is then compared to a reference signal. The reference signal in this case may simply be a signal level, which represents the body channel sensing signal amplitude as transmitted and before it has passed through the body. Alternatively, the reference signal used for comparison may be obtained during a calibration as explained above. Thus, the reference signal for comparison may represent the attenuated signal for the initial insert position, and deviations in attenuation are then determined and interpreted as new insert positions.

Thus, in a most simple implementation, the insert issues a transmit signal (in the 10 MHz to 200 MHz range) either in response to an external command or periodically. The probe then receives the signal and measures the amplitude to obtain location information. Thus, a most simple implementation does not require significant communication between the probe and insert.

A system may combine phase measurement and amplitude measurement.

The invention is explained above with reference to an example in which the implanted device is the transmitter and the external detection device is the receiver. However, the roles can be interchanged without changing the end result. Also the proposed architectures for the transmitter and the receiver can be part of a larger system in the implanted device and the external detection device. Furthermore, in the example shown, all processing functions are shown in the receiver (i.e. the probe). Some of these functions may be located remotely from the receiver (probe) in a device with which the receiver communicates, such as the database and alarm system.

The invention is of interest for detecting migration of any electronic implanted device inside the body.

As discussed above, embodiments make use of a controller in the probe and in the implanted device. The controller can be implemented in numerous ways, with software and/or hardware, to perform the various functions required. A processor is one example of a controller which employs one or more microprocessors that may be programmed using software (e.g., microcode) to perform the required functions. A controller may however be implemented with or without employing a processor, and also may be implemented as a combination of dedicated hardware to perform some functions and a processor (e.g., one or more programmed microprocessors and associated circuitry) to perform other functions.

Examples of controller components that may be employed in various embodiments of the present disclosure include, but are not limited to, conventional microprocessors, application specific integrated circuits (ASICs), and field-programmable gate arrays (FPGAs).

In various implementations, a processor or controller may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor or controller.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

## Claims

1. A system for determining the position of an implanted device in a human or animal body relative to a probe, wherein the implanted device comprises an implanted device 10 MHz to 200 MHz RF transmitter (12) or receiver, and the probe comprises a probe 10 MHz to 200 MHz RF receiver (14) or transmitter, wherein the system comprises:
a controller (22) which is adapted to determine a phase angle and/or an amplitude attenuation or difference between a reference signal and a received signal which has passed through the body and thereby determine the proximity of the probe to the implanted device.

2. A system as claimed in claim 1, comprising a communications system (26) for communication between the implanted device and the probe, wherein the implanted device comprises an implanted device clock (18) and the probe comprises a probe clock (24), wherein the controller is adapted to determine a phase angle, and wherein the communications system comprises a clock synchronization system (28) for synchronizing the probe clock (24) with the implanted device clock (18).

3. A system as claimed in claim 1 or 2, further comprising a database (68) which stores mappings between phase angles and/or amplitude attenuations and proximity distances.

4. A system as claimed in any preceding claim, further comprising an alarm system (69) for generating an alarm if a detected proximity exceeds a threshold value which depends on a proximity value corresponding to an initial placement of the implanted device.

5. A system as claimed in any preceding claim, further comprising a location determining system (67) for determining an implanted device position based on a set of at least three proximity determinations each associated with a pre-determined probe position.

6. A system as claimed in any preceding claim, wherein the system is part of the probe (12).

7. A system as claimed in any one of claims 1 to 5, wherein the system is part of the implanted device (10), and wherein the system further comprises a wireless communication system for communicating the determined proximity.

8. A system as claimed in any preceding claim, wherein the probe is an external probe.

9. A system as claimed in claim 8, wherein the system further comprises a marker (89) for application to the skin of the human or animal to identify an initial position closest to the implanted device.

10. A system as claimed in any one of claims 1 to 7, wherein the probe is an implanted device.

11. An implant system, comprising:
an implantable device (10) which comprises an implanted device 10 MHz to 200 MHz RF transmitter or receiver;
a probe (12), which comprises a probe 10 MHz to 200 MHz RF receiver or transmitter; and
a system as claimed in any preceding claim provided at the implantable device or at the probe.

12. A method for determining the position of an implanted device in a human or animal body relative to a probe, wherein the implanted device comprises an implanted device 10 MHz to 200 MHz RF transmitter or receiver, and the probe comprises a 10 MHz to 200 MHz RF receiver or transmitter, wherein the method comprises:
generating a reference signal at one of the implanted device (10) and the probe (12); and
at the other of the implanted device and the probe determining a phase angle and/or an amplitude attenuation or difference between the reference signal and a received signal; and
thereby determining the proximity of the probe to the implanted device.

13. A method as claimed in claim 12, wherein the implanted device comprises an implanted device clock (18) and the probe comprises a probe clock (24), wherein the method further comprises synchronizing the probe clock with the implanted device clock, and using a phase angle to determine the proximity.

14. A method as claimed in claim 12 or 13, comprising using stored mappings to translate between phase angles or amplitude attenuations and proximity distances.

15. A computer program product, comprising code means which is adapted, when said computer program is run on a computer, to perform the method of any one of claims 12 to 14.
